# EUROPEAN PATENT APPLICATION

(11) **EP 2 062 587 A1**
(43) Date of publication of application: **27.05.2009**
(21) Application number: 07805787.4
(22) Date of filing: 30.08.2007
(51) Int. Cl.: A61K 35/74, A23K 1/16, A23L 1/30, A61P 3/06

(54) **AGENT FOR ACCELERATING THE INCREASE IN AND/OR PREVENTING THE DECREASE IN BLOOD ADIPONECTIN LEVEL, AND VISCERAL FAT ACCUMULATION INHIBITOR**

(30) Priority: 04.09.2006 JP 2006239290; 08.09.2006 JP 2006244377
(71) Applicant: Snow Brand Milk Products Co., Ltd., Sapporo-shi Hokkaido 065-0043 (JP)
(72) Inventor: KAWAKAMI, Hiroshi, Kawagoe-shi, Saitama 350-1165 (JP); HIGURASHI, Satoshi, Kawagoe-shi, Saitama 350-1165 (JP); MATSUYAMA, Hiroaki, Kawagoe-shi, Saitama 350-1165 (JP)
(74) Representative: Noergaard, Jens Viktor
(86) International application number: PCT/JP2007/000932
(87) International publication number: WO 2008/029505

(57) **Abstract**

Disclosed is an agent for accelerating the increase in and/or preventing the decrease in the blood adiponectin level, or a food, beverage or animal feed for accelerating the increase in and/or preventing the decrease in the blood adiponectin level, which comprises a culture or cell of a lactic acid bacterium belonging to the genus *Lactobacillus,* particularly *Lactobacillus gasseri* or *Lactobacillus helveticus* as an active ingredient. Also disclosed is a visceral fat accumulation inhibitor or a food or beverage for inhibiting the accumulation of a visceral fat, which comprises a culture or cell of *Lactobacillus helveticus* as an active ingredient.

## Description

### TECHNICAL FIELD

The present invention relates to a blood-adiponectin-concentration increase accelerator and/or decrease inhibitor containing a culture and/or cells itself of lactic acid bacteria of the genus *Lactobacillus* (lactobacilli) (particularly *Lactobacillus gasseri* or *Lactobacillus helveticus*) as an active ingredient, a novel food or drink having a blood-adiponectin-concentration increase accelerating- and/or decrease inhibiting-effect, and a novel feed having a blood-adiponectin-concentration increase accelerating- and/or decrease inhibiting effect. The blood-adiponectin-concentration-increase accelerator and/or decrease inhibitor according to the present invention can accelerate the increase in blood-adiponectin-concentration and/or suppress the decrease in blood-adiponectin-concentration upon ingestion. The decrease in blood-adiponectin-concentration is considered to cause metabolic syndrome that may result in cardiovascular disease (e.g., thrombosis, insulin resistance, abnormal glucose metabolism, or hypertension). The present invention is effective for preventing or treating the metabolic syndrome.
The present invention also relates to a visceral fat accumulation inhibitor containing a culture and/or cells itself of *Lactobacillus helveticus* (i.e., lactic acid bacteria of the genus *Lactobacillus*) as an active ingredient, and a novel food or drink having a visceral fat accumulation-inhibition effect. The visceral fat accumulation inhibitor and the visceral fat accumulation inhibiting food or drink according to the present invention can inhibit visceral fat accumulation upon ingestion. Visceral fat accumulation is considered to cause metabolic syndrome that may result in cardiovascular disease (e.g., thrombosis, insulin resistance, abnormal glucose metabolism, or hypertension). The present invention is effective for preventing or treating the metabolic syndrome.

### BACKGROUND ART

In recent years, the number of patients suffering from lifestyle-related disease such as diabetes, hypertension, hyperlipemia, or arteriosclerosis has increased along with westernization of lifestyle. In particular, cardiovascular diseases and cerebrovascular diseases are responsible for about one-third of all deaths, and the number of deaths caused by cardiovascular diseases and cerebrovascular diseases has increased year by year. Therefore, measures against cardiovascular diseases and cerebrovascular diseases have become an important issue. The risk of these atherosclerotic diseases increases significantly due to a combination of risk factors such as hypertension, hyperlipemia, and glucose intolerance. A combination of such risk factors has been widely recognized as the metabolic syndrome. According to a study conducted on 120,000 Japanese workers, a person who has one of the risk factors "obesity", "hypertension", "hyperglycemia", "hypertriglyceridemia (hyperlipidemia)", and "hypercholesterolemia" has five (5) times higher the risk of developing heart disease, a person who has two of the risk factors has ten (10) times higher the risk of developing heart disease, and a person who has three or four of the risk factors has thirty one (31) times higher the risk of developing heart disease, irrespective of the degree of severity of each of the risk factors. The Ministry of Health, Labour and Welfare of Japan reported that the number of patients suffering from hypertension is 39,000,000, the number of patients suffering from hyperlipemia is 22,000,000, the number of patients suffering from diabetes (including would-be patients) is 16,200,000, and the number of patients suffering from obesity is 4,680,000. The number of patients suffering from these diseases has increased year by year.

The term "metabolic syndrome" refers to "multiple risk factor syndrome that is characterized by visceral fat accumulation and at least one of: insulin resistance, abnormal glucose metabolism, lipid metabolism disorder, and hypertension due to visceral fat accumulation and may result in arteriosclerosis at high risk". The decrease in blood-adiponectin-concentration due to visceral fat accumulation is a fundamental factor of the metabolic syndrome and arteriosclerosis. A fat tissue (i.e., a secretory tissue predominant in a living body) produces various endocrine factors, and is involved in the maintenance of homeostasis of a living body. However, excessive visceral fat accumulation breaks down the secretion balance among the endocrine factors and causes various pathological conditions. In particular, production of the endocrine factors (e.g., plasminogen activator inhibitor (PAI-1), tumor necrosis factor (TNF-α), and leptin) increases due to visceral fat accumulation so that thrombosis, insulin resistance, abnormal glucose metabolism, hypertension, and the like occur.

As a means for reducing the amount of visceral fat, exercise therapy, diet therapy, behavioral therapy, and drug therapy are employed. Regarding the drug therapy, a centrally-acting appetite suppressant has been developed. A β3-adrenoceptor stimulant, a drug that inhibits lipid absorption through the digestive tract, and the like, have also been developed. However, the efficacy of such an anti-obesity agent on the decrease in the amount of visceral fat (particularly visceral fat around the kidney) has not been confirmed, and drug therapy effective for decreasing the amount of visceral fat around the kidney has not been established. Moreover, diet therapy that reduces the amount of visceral fat by ingestion of daily meal or supplements has not been established.

Adiponectin that is specifically secreted by fat tissue is normally contained in blood at a high level. The adiponectin level decreases due to visceral fat accumulation. Adiponectin has various physiological functions such as preventing diabetes, arteriosclerosis, inflammation, and hypertension. It is very important to accelerate an increase in blood-adiponectin-concentration or suppress the decrease in blood-adiponectin-concentration in order to prevent or treat the metabolic syndrome.

Drug therapy has been employed as measures to treat each pathological condition involved in the metabolic syndrome. As a drug therapy centrally-acting appetite suppressant, a β3-adrenoceptor stimulant, a drug that suppresses lipid absorption through the digestive tract, and the like have been developed. However, the efficacy of such an anti-obesity agent on the decrease in the amount of visceral fat has not been confirmed, and drug therapy effective for decreasing the amount of visceral fat has not been established. Moreover, it has been a problem that such an agent requires a prescription and causes side effects. Even if one pathological condition is treated, more serious pathological condition may develop due to other pathological conditions. Therefore, it is necessary to regulate the balance among the upstream endocrine factors derived from the adipocyte. Accordingly, a change in lifestyle is considered to be important (i.e., exercise therapy or diet therapy rather than drug therapy) in order to prevent or treat the metabolic syndrome caused by visceral fat accumulation. Therefore, a food or drink that is effective for preventing or treating the metabolic syndrome caused by visceral fat accumulation and can be taken safely and daily over a long period of time has been longed for.

As a substance that accelerates an increase in blood-adiponectin-concentration, plant extracts such as an apple extract (e.g., see Patent Document 1), a hop bract extract (e.g., see Patent Document 2), green tea catechin (e.g., see Patent Document 3), a rice bran extract (e.g., see Patent Document 4), and a *Curcumae Rhizoma* extract (e.g., see Patent Document 5) have been disclosed. However, since these substances may require complicated extraction conditions, or the availability of the raw material may be limited, it may be difficult to stably supply a raw material for a preparation, food, or drink. It is not known that a culture or cells of bacteria have an effect of accelerating the increase in adiponectin level or suppressing the decrease in adiponectin level.

A visceral fat accumulation preventive composition or an obesity treatment composition containing a culture of fungi such as *Aspergillus oryzae*, a culture of lactic acid bacteria such as *Streptococcus faecalis*, and yeast have been disclosed (e.g., see Patent Document 6). However, since this method mixes the three raw materials individually cultured, the production process becomes complicated. On the other hand, since the present invention utilizes a culture or cells of single bacteria, the production process is facilitated.

*Lactobacillus gasseri* has a pathogen infection-protection effect (e.g., see Patent Document 7), an inflammatory bowel disease- or irritable bowel syndrome-prevention effect (e.g., see Patent Document 8), a diabetes complication-prevention effect (e.g., see Patent Document 9), a serum-cholesterol-elevation inhibition effect (e.g., see Patent Document 10), a bone resorption-inhibition effect (e.g., see Patent Document 11), an immunostimulant effect (e.g., see Patent Document 12), and the like. However, it is not known that a fermented product or cells itself of *Lactobacillus gasseri* have a blood-adiponectin-concentration increase-acceleration- or decrease-inhibition-effect.

*Lactobacillus helveticus* has been typically used as a lactic acid bacteria starter for dairy products from old times. *Lactobacillus helveticus has strong proteolytic activity, and produces an extracellular proteinase which has very high activity and plays an important role with regard to milk fermentability*. Specifically, the extracellular proteinase decomposes milk proteins and produces various peptide fragments. The peptides thus produced are hydrolyzed due to peptidases to have reduced molecular weights. Peptides produced in a culture medium due to proteolytic enzymes are partially incorporated in lactic acid bacteria cells and are utilized as a nitrogen source. Some peptides produced in a culture medium inhibit an angiotensin-converting enzyme (hereinafter referred to as "ACE") (i.e., a substance that increases blood pressure) (e.g., see Non-patent Document 1). As peptides that inhibit ACE enzyme activity to suppress hypertension, a number of effective peptides have been found in milk proteins, soybean proteins, fish protein degradation products, and the like. Peptides having ACE inhibitor activity contained in fermented milk produced using *Lactobacillus helveticus* are Val-Pro-Pro and Ile-Pro-Pro, and it has been confirmed that these lactotripeptides have a strong hypotensive effect by an experiment using spontaneously hypertensive rats (SHR) (e.g., see Non-patent Document 2).
It has been suggested that lactotripeptide has a stress relaxation effect in addition to hypotensive activity (e.g., see Patent Documents 13 and 14). *Lactobacillus helveticus* also has immunostimulatory activity (e.g., see Patent Document 15). However, it is not known that a fermented product or cells itself of *Lactobacillus helveticus* have a blood-adiponectin-concentration increase-acceleration- or decrease-inhibition-effect. It is not known that a culture and/or cells itself of *Lactobacillus helveticus* have a visceral fat accumulation-inhibition effect and a visceral fat decrease acceleration effect.
Patent Document 1: JP-A-2006-193502
Patent Document 2: JP-A-2006-193501
Patent Document 3: JP-A-2006-131512
Patent Document 4: JP-A-2005-68132
Patent Document 5: JP-A-2005-60308
Patent Document 6: JP-A-2004-99539
Patent Document 7: JP-A-8-268899
Patent Document 8: JP-A-2003-95963
Patent Document 9: JP-A-2003-252770
Patent Document 10: JP-A-2003-306436
Patent Document 11: JP-A-2004-315477
Patent Document 12: JP-A-2006-69993
Patent Document 13: JP-A-10-45610
Patent Document 14: JP-A-11-98978
Patent Document 15: JP-A-2006-76961
Non-patent Document 1: J. Dairy Sci., 78: pp. 777 to 783 (1995)
Non-patent Document 2: J. Dairy Sci., 78: pp. 1253 to 1257 (1995)

### DISCLOSURE OF THE INVENTION

### PROBLEMS TO BE SOLVED BY THE INVENTION

An object of the present invention is to provide a blood-adiponectin-concentration increase-acceleration and/or decrease-inhibition agent that can be taken daily, accelerates an increase in blood-adiponectin-concentration and/or suppresses the decrease in blood-adiponectin-concentration upon ingestion, and is effective for preventing or treating the metabolic syndrome, as well as a food, drink, and feed having such a function.
Another object of the present invention is to provide a visceral fat accumulation inhibitor that can be taken daily, suppresses visceral fat accumulation upon ingestion, and is effective for preventing or treating the metabolic syndrome, and a food or drink having such a function.

### MEANS FOR SOLVING THE PROBLEMS

The inventors of the present invention extensively searched for a milk component that suppresses the decrease in blood-adiponectin-concentration which is said to increase the risk of cardiovascular disease. Consequently, the inventors found that a culture or cells itself of lactic acid bacteria of the genus *Lactobacillus* (particularly *Lactobacillus gasseri* or *Lactobacillus helveticus*) have an extremely high blood-adiponectin-concentration increase-acceleration and/or decrease-inhibition effect. This finding has led to the completion of the present invention.
The inventors also extensively searched for a bacterial species that decreases visceral fat said to cause the metabolic syndrome from among various microorganisms. As a result, the inventors found that, among lactic acid bacteria of *Lactobacillus* genus, a culture and/or cells itself of *Lactobacillus helveticus* have an extremely high visceral fat accumulation-inhibition effect. This finding has led to the completion of the present invention.

Specifically, the present invention provides the following.
(1) A blood-adiponectin-concentration increase-acceleration and/or decrease-inhibition agent comprising a culture and/or cells itself of lactic acid bacteria of the genus *Lactobacillus* as an active ingredient.
(2) The blood-adiponectin-concentration increase-acceleration and/or decrease-inhibition agent according to (1), wherein the lactic acid bacteria of the genus *Lactobacillus* are *Lactobacillus gasseri* or *Lactobacillus helveticus.*
(3) A blood-adiponectin-concentration increase-acceleration and/or decrease-inhibition food or drink comprising a culture and/or cells itself of lactic acid bacteria of the genus *Lactobacillus* as an active ingredient.
(4) The blood-adiponectin-concentration increase-acceleration and/or decrease-inhibition food or drink according to (3), wherein the lactic acid bacteria of the genus *Lactobacillus* are *Lactobacillus gasseri* or *Lactobacillus helveticus.*
(5) A blood-adiponectin-concentration increase-acceleration and/or decrease-inhibition feed comprising a culture and/or cells itself of lactic acid bacteria of the genus *Lactobacillus* as an active ingredient.
(6) The blood-adiponectin-concentration increase-acceleration and/or decrease-inhibition feed according to (5), wherein the lactic acid bacteria of the genus *Lactobacillus* are *Lactobacillus gasseri* or *Lactobacillus helveticus.*
(7) A visceral fat accumulation inhibitor comprising a culture and/or cells itself of *Lactobacillus helveticus* as an active ingredient.
(8) A visceral fat accumulation-inhibition food or drink comprising a culture and/or cells itself of *Lactobacillus helveticus* as an active ingredient.

### EFFECT OF THE INVENTION

The blood-adiponectin-concentration increase-acceleration and/or decrease-inhibition agent and the blood-adiponectin-concentration increase-acceleration and/or decrease-inhibition food, drink, or feed according to the present invention are useful for preventing or treating the metabolic syndrome which is said to be caused by the decrease in blood-adiponectin-concentration. Since the blood-adiponectin-concentration increase-acceleration and/or decrease-inhibition agent and the blood-adiponectin-concentration increase-acceleration and/or decrease-inhibition food, drink, or feed according to the present invention utilize a culture or cells itself of lactic acid bacteria of the genus *Lactobacillus* (particularly *Lactobacillus gasseri* or *Lactobacillus helveticus*), a large amount of products can be supplied at relatively low cost. Moreover, these products are highly safe.

The visceral fat accumulation inhibitor and the visceral fat accumulation-inhibition food or drink according to the present invention containing a culture and/or cells itself of *Lactobacillus helveticus* as an active ingredient are useful for preventing or treating the metabolic syndrome said to be caused by visceral fat accumulation. Since the visceral fat accumulation inhibitor and the visceral fat accumulation-inhibition food or drink according to the present invention utilize a culture and/or cells itself of *Lactobacillus helveticus*, a large amount of products can be supplied at relatively low cost. Moreover, these products have a property of being highly safe due to the absence of toxicity and side effects. The visceral fat accumulation inhibitor and the visceral fat accumulation-inhibition food or drink according to the present invention can thus be taken daily as a visceral fat accumulation-inhibition supplement, food, or drink. Therefore, the visceral fat accumulation inhibitor and the visceral fat accumulation-inhibition food or drink according to the present invention are very useful.

### BEST MODE FOR CARRYING OUT THE INVENTION

The inventors have selected strains, from numerous lactic acid bacteria of fermented milk or human origin, that exhibit high stomach acid resistance, a high growth rate under low pH conditions, a high degree of intestinal colonization, affinity to human intestinal cells, and bile acid resistance, and have high survival properties, an excellent flavor, and excellent properties when applied to food. The inventors found that lactic acid bacteria of the genus *Lactobacillus* (particularly *Lactobacillus gasseri* or *Lactobacillus helveticus*) have an effect of accelerating the increase in blood-adiponectin-concentration and/or suppressing the decrease in blood-adiponectin-concentration among the lactic acid bacteria that exhibit a high degree of intestinal colonization in humans. As strains of *Lactobacillus gasseri* that satisfy the above-mentioned conditions, the inventors selected *Lactobacillus gasseri* SBT2055, *Lactobacillus gasseri* JCM1131, and *Lactobacillus gasseri* ATCC19992. *Lactobacillus gasseri* SBT2055 is deposited with the International Patent Organism Depositary, National Institute of Advanced Industrial Science and Technology (deposit number: FERM P-15535). *Lactobacillus gasseri* JCM1131 is available from RIKEN, Japan, and *Lactobacillus gasseri* ATCC19992 is available from the American Type Culture Collection.

In particular, *Lactobacillus gasseri* SBT2055 exhibits high affinity to human intestinal cells, can reach the intestine without becoming extinct when orally administered, can reside in the intestine for a long period of time, is grown in the intestine to act on the host, and exhibits an excellent effect of accelerating the increase in blood-adiponectin-concentration and/or suppressing the decrease in blood-adiponectin-concentration. It has not previously known that a strain of *Lactobacillus gasseri* administered from the outside of the body colonizes the intestine and exhibits such a physiological effect. In the present invention, other viable or dead *Lactobacillus gasseri* isolated from human or fermented milk may also be used insofar as the above-mentioned effect can be achieved. A variant of *Lactobacillus gasseri* which exhibits the above-mentioned effect may also be used. As a culture medium for *Lactobacillus gasseri* according to the present invention, various culture media such as a milk culture medium, a culture medium containing a milk component, and a semi-synthetic medium that does not contain a milk component may be used. Examples of such culture media include a reconstituted skim milk medium prepared by dissolving skim milk and sterilizing the solution by heating.

As strains of *Lactobacillus helveticus* that exhibit a blood-adiponectin-concentration increase-acceleration and/or decrease-inhibition effect and satisfy the above-mentioned conditions and strains effective for suppressing visceral fat accumulation, the inventors selected *Lactobacillus helveticus* SBT2171, *Lactobacillus helveticus* ATCC10386, and *Lactobacillus helveticus* ATCC10797. In this case, the effect of the present invention is expected to be achieved by viable or dead cells. *Lactobacillus helveticus* SBT2171 is deposited with the International Patent Organism Depositary, National Institute of Advanced Industrial Science and Technology (deposit number: FERM P-14381). *Lactobacillus helveticus* ATCC10386 and *Lactobacillus helveticus* ATCC 10797 are available from the American Type Culture Collection.

The above-mentioned lactic acid bacteria of the genus *Lactobacillus* are cultured by stationary culture or neutral culture in which the pH is controlled at a constant value. Note that the culture method is not particularly limited insofar as the lactic acid bacteria are grown advantageously. Cells cultured using a normal lactic acid bacteria culture method and isolated from the resulting culture by centrifugation or the like may be used directly as the active ingredient according to the present invention. A culture, a fermented product, a suspension, other cell-containing products, or cytoplasms or cell wall fractions obtained by treating cells using an enzyme or a physical means may also be used instead of the isolated cells. When using cells itself as the active ingredient, viable or dead cells may be used.

The blood-adiponectin-concentration increase-acceleration and/or decrease-inhibition agent and the visceral fat accumulation inhibitor according to the present invention includes a culture and/or cells itself of lactic acid bacteria of the genus *Lactobacillus* as the active ingredient. When preparing the blood-adiponectin-concentration increase-acceleration and/or decrease-inhibition agent, a pharmaceutically acceptable vehicle, stabilizer, flavoring agent, and the like may be appropriately mixed with the culture and/or cells of the lactic acid bacteria, and the mixture may be concentrated and freeze-dried. Alternatively, the lactic acid bacteria may be killed by drying with heating. The blood-adiponectin-concentration increase-acceleration and/or decrease-inhibition agent may be a dried product, a concentrate, or a paste. A vehicle, a binder, a disintegrator, a lubricant, a flavoring agent, a suspending agent, a coating agent, or any other agents may be mixed insofar as the effect of the cells or the culture is not impaired. The dosage form may be a tablet, a pill, a capsule, a granule, a powder, a syrup, or the like. As the administration method, oral administration is preferable.

The present invention also provides a food or drink including a fermented product or cells itself of lactic acid bacteria of the genus *Lactobacillus* (particularly *Lactobacillus gasserior Lactobacillus helveticus*), as an active ingredient, and having a blood-adiponectin-concentration increase-acceleration and/or decrease-inhibition effect, and a visceral fat accumulation-inhibition food or drink including a fermented product or cells itself of *Lactobacillus helveticus.*

The food or drink having a blood-adiponectin-concentration increase-acceleration and/or decrease-inhibition effect according to the present invention may be a culture, a fermented product, or cells itself of lactic acid bacteria of the genus *Lactobacillus* (particularly *Lactobacillus gasseri* or *Lactobacillus helveticus*), or may be a food or drink containing cells, a culture, or a fermented product of lactic acid bacteria of the genus *Lactobacillus* (particularly *Lactobacillus gasseri* or *Lactobacillus helveticus*).

The visceral fat accumulation-inhibition food or drink may be a culture, a fermented product, or cells itself of *Lactobacillus helveticus*, or may be a food or drink containing cells, a culture, or a fermented product of *Lactobacillus helveticus.*

As the culture or the fermented product, a fermented food such as yogurt or cheese is suitable. The cells, culture, or fermented product may be mixed into any food or drink, or may be added to a raw material when producing a food or drink. Examples of the food or drink include milk drinks, fermented milk, fruit drinks, jelly, candy, dairy products, egg products such as mayonnaise, cake and bread such as butter cake, and the like. The cells, culture, or fermented product may be mixed into dried milk or a nourishment composition aimed at babies, infants, low birth-weight infants, and the like. When using the lactic acid bacteria as viable cells, bread, snack, cake, pudding, or the like for blood-adiponectin-concentration increase-acceleration and/or decrease-inhibition or visceral fat accumulation-inhibition may be produced by using, as a raw material, cells itself of lactic acid bacteria of the genus *Lactobacillus* (particularly *Lactobacillus gasseri* or *Lactobacillus helveticus*) and fermented milk or cheese obtained by fermentation of cells of the lactic acid bacteria above. Since such a product can be ingested daily and has a blood-adiponectin-concentration increase-acceleration and/or decrease-inhibition effect or a visceral fat accumulation-inhibition effect, such a product is effective for preventing or treating the metabolic syndrome caused by the decrease in blood-adiponectin-concentration or visceral fat accumulation.

The present invention also provides a feed including a fermented product or cells itself of lactic acid bacteria of the genus *Lactobacillus* as an active ingredient (particularly *Lactobacillus gasseri* or *Lactobacillus helveticus*) and having a blood-adiponectin-concentration increase-acceleration and/or decrease-inhibition effect or a visceral fat accumulation-inhibition effect. The cells, culture, or fermented product may be mixed into any animal feed, or may be added to a raw material when producing the feed.

In the present invention, in order to achieve a blood-adiponectin-concentration increase-acceleration and/or decrease-inhibition effect or a visceral fat accumulation-inhibition effect, the amount of lactic acid bacteria and the like may be adjusted so that an adult can absorb 10 to 200 g of a culture of *Lactobacillus gasseri* and/or *Lactobacillus helveticus* or 0.1 to 5000 mg of cells itself of *Lactobacillus gasseri* and/or *Lactobacillus helveticus* per day. The content ratio of the lactic acid bacteria is not particularly limited, but may be appropriately adjusted depending on the ease of production, a preferable daily dosage, and the like. For example, when employing a liquid dosage form, the content of the lactic acid bacteria is preferably 1×10⁵ cells/ml to 1×10¹⁰ cells/ml. When employing a solid dosage form, the content of the lactic acid bacteria is preferably 1×10⁵ cells/g to 1×10¹⁰ cells/g. When administering viable lactic acid bacteria, the effect of the present invention can be achieved by administering the lactic acid bacteria in an amount of 10⁸ to 10¹² cfu/day (adult). The lactic acid bacteria colonize the intestine upon administration and exhibit the desired effect.

Since the blood-adiponectin-concentration increase-acceleration and/or decrease-inhibition agent or the blood-adiponectin-concentration increase-acceleration and/or decrease-inhibition food, drink, or feed have a blood-adiponectin-concentration increase-acceleration and/or decrease-inhibition effect, the agent, food, drink, or feed are useful for preventing, treating, or improving various pathological conditions caused by the decrease in blood-adiponectin-concentration.
Since the visceral fat accumulation inhibitor, food, or drink according to the present invention have a visceral fat accumulation-inhibition effect, the inhibitor, food, or drink are useful for preventing, treating, or improving various pathological conditions caused by visceral fat accumulation.
The lactic acid bacteria used in the present invention have been utilized for producing fermented milk and cheese from old times. Therefore, the blood-adiponectin-concentration increase-acceleration and/or decrease-inhibition agent, the blood-adiponectin-concentration increase-acceleration and/or decrease-inhibition food, drink, or feed, and the visceral fat accumulation inhibitor, food, or drink according to the present invention are safe.

The present invention is further described below by way of examples and test examples. Note that the following examples should not be construed as limiting the present invention.

### EXAMPLE 1

### (Preparation 1 of Lactobacillus gasseri culture powder)

A reconstituted skim milk medium (contains powdered skim milk: 13 wt%, yeast extract: 0.5 wt%) was sterilized at 95°C for 30 minutes. *Lactobacillus gasseri* SBT2055 was then inoculated into the medium, and cultured at 37°C for 16 hours. The resulting culture was freeze-dried to obtain a culture powder of *Lactobacillus gasseri* SBT2055. The culture powder can be used directly as a blood-adiponectin-concentration increase-acceleration and/or decrease-inhibition agent according to the present invention.

### EXAMPLE 2

### (Preparation 2 of Lactobacillus gasseri culture powder)

A reconstituted skim milk medium (contains powdered skim milk: 13 wt%, yeast extract: 0.5 wt%) was sterilized at 95°C for 30 minutes. *Lactobacillus gasseri* JCM1131 was then inoculated into the medium, and cultured at 37°C for 16 hours. The resulting culture was freeze-dried to obtain a culture powder of *Lactobacillus gasseri* JCM1131. The culture powder can be used directly as the blood-adiponectin-concentration increase-acceleration and/or decrease-inhibition agent according to the present invention.

### [Test Example 1]

### (Administration of Lactobacillus gasseri culture and measurement of blood-adiponectin-concentration)

A control food was fed, for one week, to fifty Fischer male rats of four-week-old. The rats were then divided equally into five groups. The control food was fed to one group for four weeks. A test food containing *Lactobacillus gasseri* SBT2055, a test food containing *Lactobacillus gasseri* JCM1131, a test food containing *Lactobacillus gasseri* ATCC19992, and a test food containing *Lactobacillus casei* ATCC11578 were respectively fed to the remaining four groups for four weeks. The blood-adiponectin-concentrations of the rats were then measured. No difference in weight, food intake, and excrement weight was observed for the five groups.
Table 1 shows the blood-adiponectin-concentration measurement results. As shown in Table 1, the groups which were fed the test food containing *Lactobacillus gasseri* SBT2055, the test food containing *Lactobacillus gasseri* JCM1131, the test food containing *Lactobacillus gasseri* ATCC19992, and the test food containing *Lactobacillus casei* ATCC11578 showed significantly high blood-adiponectin-concentrations as compared with the control food group. Specifically, it was confirmed that the food containing the culture powder of *Lactobacillus gasseri* SBT2055, *Lactobacillus gasseri* JCM1131, *Lactobacillus gasseri* ATCC19992, or *Lactobacillus casei* ATCC11578 maintained a constant blood-adiponectin-concentration.

**TABLE 1**

| Change in blood adiponectin concentration | | | |
|---|---|---|---|
| Feed groups | Blood-adiponectin-concentration (µg/ml) | | Change rate (%) |
| | Four weeks | Eight weeks | |
| Control food | 11.01 | 10.27 | 93.3 |
| SBT2055 containing food | 10.93 | 15.28 | 139.8 |
| JCM1131 cont. food | 11.22 | 13.98 | 124.6 |
| ATCC19992 cont. food | 11.34 | 13.55 | 119.5 |
| ATCC11578 cont. food | 12.33 | 14.07 | 114.1 |

As is clear from the above results, it was confirmed that the food containing the culture powder of *Lactobacillus gasseri* suppressed the decrease in blood-adiponectin-concentration, which may increase the risk of cardiovascular disease, and maintained the blood-adiponectin-concentration at an appropriate value.

### EXAMPLE 3

### (Production of tablet)

A liquid culture of *Lactobacillus gasseri* SBT2055 was centrifuged (7000 rpm) at 4°C for 15 minutes, and washed with sterilized water. This procedure was repeated three times to obtain washed cells. The washed cells were freeze-dried to obtain a cell powder. Four (4) parts of powdered skim milk was mixed with one (1) part of the cell powder. The mixed powder was formed into tablets (1 g) by a normal method using a tableting machine to obtain tablets each containing 200 mg of the cells itself of *Lactobacillus gasseri* SBT2055 according to the present invention.

### EXAMPLE 4

### (Production of fermented milk)

*Lactobacillus gasseri* SBT2055 was cultured in an MRS liquid medium (manufactured by Difco). The culture solution in a logarithmic growth phase was inoculated in an amount of 1% into 10% reconstituted skim milk (sterilized at 115°C for 20 minutes) to which a yeast extract was added in an amount of 0.3% to prepare a mother culture. Ten (10) % reconstituted skim milk was then added to the mother culture. The mixture was added to a yogurt mixture heated at 100°C for 10 minutes in an amount of 2.5%. The mixture was fermented at 37°C, and was cooled when the lactic acidity reached 0.85 to terminate fermentation to obtain blood-adiponectin-concentration increase-acceleration and/or decrease-inhibition fermented milk according to the present invention.

### EXAMPLE 5

### (Production of powder)

*Lactobacillus gasseri* SBT2055 was inoculated into an MRS liquid medium (5L) (manufactured by Difco), and was then subjected to stationary culture at 37°C for 18 hours. The culture was then centrifuged (7000 rpm) for 15 minutes to obtain concentrated cells (1/50th of the volume of the culture solution). The concentrated cells were mixed with a dispersion medium containing 10 wt% of powdered skim milk and 1 wt% of monosodium glutamate, in an amount equal to that of the dispersion medium. After adjusting the pH of the mixture to 7.0, the mixture was freeze-dried. The resulting freeze-dried product was filtered through a 60-mesh sieve to prepare freeze-dried cells. Four hundred (400) g of lactose (JP) and 600 g of potato starch (JP) were added to 1 g of the freeze-dried cells in accordance with the "Powder" section of Japanese Pharmacopoeia Thirteenth Edition, and the components were uniformly mixed to obtain a blood-adiponectin-concentration increase-acceleration and/or decrease-inhibition powder according to the present invention.

### EXAMPLE 6

### (Production of stick health food)

Forty (40) g of a mixture of vitamin C and citric acid (equal quantity), 100 g of granulated sugar, and 60 g of a mixture of cornstarch and lactose (equal quantity) were added to 30 g of the culture powder of *Lactobacillus gasseri* SBT2055 obtained in Example 1. A stick shape bag was charged with the mixture to obtain a blood-adiponectin-concentration increase-acceleration and/or decrease-inhibition stick health food according to the present invention.

### EXAMPLE 7

### (Production of natural cheese)

Raw material milk, of which the fat percentage was adjusted, was subjected to plate pasteurization at 75°C for 15 seconds. After cooling the raw material milk to 30°C, calcium chloride (0.01 %) was added. A commercially available lactic acid bacteria starter (manufactured by Christian Hansen) (0.7%) and *Lactobacillus gasseri* SBT2055 (1%) were added to the raw material milk. After the addition of rennet (0.003%) to coagulate the milk, the resulting product was cut. The cut product was stirred until the pH reached 6.1 to 6.2, and whey was discharged to obtain curds. The curds were placed in a mold and then compressed. Salt was then added to the resulting product to obtain blood-adiponectin-concentration increase-acceleration and/or decrease-inhibition natural cheese according to the present invention.

### EXAMPLE 8

### (Production of capsule)

Raw materials were mixed according to the formulation shown in Table 2 and then granulated. A capsule was charged with the resulting product to obtain a blood-adiponectin-concentration increase-acceleration and/or decrease-inhibition capsule according to the present invention.

**TABLE 2**

| | |
|---|---|
| SBT2055 (Example 1) | 20.0 (wt%) |
| Lactose | 24.5 |
| Soluble starch | 55.0 |
| Magnesium stearate | 0.5 |

### EXAMPLE 9

### (Production of drink)

Raw materials were mixed according to the formulation shown in Table 2. After charging a container with the mixture, the mixture was sterilized by heating to obtain a blood-adiponectin-concentration increase-acceleration and/or decrease-inhibition drink according to the present invention.

**TABLE 3**

| | |
|---|---|
| SBT2055 (Example 1) | 2.5 (wt%) |
| Sugar | 7.5 |
| Citric acid | 0.6 |
| Apple juice | 10.0 |
| Water | 79.4 |

### EXAMPLE 10

### (Production of low-fat hard natural cheese)

Several types of low-fat hard natural cheese were produced using raw material milk modified such that the fat percentage of the resulting cheese was 12 to 30%. Specifically, the raw material milk, of which the fat percentage was adjusted, was subjected to plate pasteurization at 75°C for 15 seconds. After cooling the raw material milk to 30°C, calcium chloride (0.01%) was added. A lactic acid bacteria starter (manufactured by Christian Hansen) (0.7%) and *Lactobacillus helveticus* SBT2171 (1%) were added to the raw material milk. After the addition of rennet (0.003%) to coagulate the milk, the resulting product was cut. The cut product was stirred until the pH reached 6.1 to 6.2, and whey was discharged to obtain curds. The curds were placed in a mold and then compressed. Salt was then added to the resulting product to obtain blood-adiponectin-concentration increase-acceleration and/or decrease-inhibition Gouda-type low-fat hard natural cheese according to the present invention.

### EXAMPLE 11

### (Production of fermented milk)

Fermented milk was produced using *Lactobacillus helveticus* SBT2171. *Lactobacillus helveticus* SBT2171 was cultured at 37°C for 12 hours using 100 g of skim milk, inoculated into a fresh medium of the same kind (3 kg), and cultured at 37°C for 12 hours. One hundred (100) kg of skim milk was fermented at 32°C for 20 hours using the resulting milk as a starter to obtain blood-adiponectin-concentration increase-acceleration and/or decrease-inhibition fermented milk according to the present invention. The viable cell count of *Lactobacillus helveticus* SBT2171 was 8.2×10⁸ cells/g.

### EXAMPLE 12

### Production of yogurt drink

Four (4) kg of granulated sugar, 3 kg of water, and 0.15 kg of pectin were added to 43 kg of the fermented milk obtained in Example 11. The mixture was then homogenized to obtain 50 kg of a yogurt drink. The yogurt drink had a mild favorable flavor and a pH of 3.6. The viable cell count of *Lactobacillus helveticus* SBT2171 was 4.6×10⁸ cells/g.

### EXAMPLE 13

### (Preparation of cells)

Five (5) kg of water was added to 5 kg of fermented milk obtained in the same manner as in Example 11. The mixture was centrifuged (3500×g) for 20 minutes using a continuous centrifuge, and only cells were collected. After the addition of 1 kg of water, the mixture was centrifuged. This procedure was repeated three times to remove non-cell components that were contained in the precipitate. Twenty (20) g of cells itself of *Lactobacillus helveticus* SBT2171 were thus collected. The cells can be used directly as a blood-adiponectin-concentration increase-acceleration and/or decrease-inhibition agent according to the present invention.

### [Test Example 2]

### (Confirmation on blood-adiponectin-concentration increase-acceleration and/or decrease-inhibition effect)

A blood-adiponectin-concentration increase-acceleration and/or decrease-inhibition effect was checked using cells itself of *Lactobacillus helveticus.* Four-week-old Fischer male rats were used for an animal experiment (one group consists of eight rats). Feed containing *Lactobacillus helveticus* were fed to groups (cell feed groups), and a feed that did not contain cells was fed to another group (control feed group). The control feed was fed to each group for four weeks, and the control feed or the feed containing *Lactobacillus helveticus* were then fed to each group for four weeks. The blood was collected in the fourth week and in the eighth week, and the blood-adiponectin-concentration was measured using a mouse/rat adiponectin ELISA kit (manufactured by Otsuka Pharmaceutical Co., Ltd.).
The results are shown in Table 4. As shown in Table 4, while the blood-adiponectin-concentration of the control feed group decreased with the passage of time, the blood-adiponectin-concentration of the cell feed group increased with the passage of time. Specifically, it was confirmed that an increase in blood-adiponectin-concentration was accelerated or the decrease in blood-adiponectin-concentration was suppressed by ingestion of *Lactobacillus helveticus.*

**TABLE 4**

| Change in blood adiponectin concentration | | | |
|---|---|---|---|
| Feed groups | Blood-adiponectin-concentration (µg/ml) | | Change rate (%) |
| | Four weeks | Eight weeks | |
| Control food | 12.21 | 11.77 | 96.4 |
| SBT2171 cell containing food | 11.98 | 14.95 | 124.8 |
| ATCC10386 c.c. food | 11.55 | 13.78 | 119.3 |
| ATCC10797 c.c. food | 12.54 | 14.34 | 114.3 |

### [Test Example 3]

### (Confirmation on adiponectin increase-acceleration and/or decrease-inhibition effect)

An adiponectin increase-acceleration and/or decrease-inhibition effect was checked using the cheese obtained in Example 10. Four-week-old Fischer male rats were used for an animal experiment (one group consists of eight rats). A feed containing the cheese was fed to one group (cheese feed group), and a feed that did not contain the cheese was fed to the other group (control feed group). The control feed contained milk casein as a protein source and a butter oil as a lipid source. The control feed was prepared so that the content of common components, main minerals, and vitamin E (α-tocopherol) was the same as that of the feed containing the cheese based on the component analysis results for the cheese (Table 5). In the feed containing the cheese, proteins and lipids other than the cheese component were not used.
The control feed was fed to each group for four weeks, and the control feed or the feed containing the cheese was then fed to each group for four weeks. The blood was collected in the fourth week and the eighth week, and the blood-adiponectin-concentration was measured using a mouse/rat adiponectin ELISA kit (manufactured by Otsuka Pharmaceutical Co., Ltd.).

**TABLE 5**

| Cheese component | | |
|---|---|---|
| Protein | | 44.8 |
| Fat | | 43.4 |
| Ash | g/100 g | 6.3 |
| Sodium | | 1050 |
| Potassium | | 130 |
| Calcium | | 1190 |
| Magnesium | | 41 |
| Phosphorus | | 850 |
| Iron | mg/100 g | 0.22 |
| α-tocopherol | mg/100 g | 0.9 |

The results are shown in Table 6. As shown in Table 6, while the blood-adiponectin-concentration of the control feed group decreased with the passage of time, the blood-adiponectin-concentration of the cheese feed group increased with the passage of time. Specifically, it was confirmed that an increase in blood-adiponectin-concentration was accelerated or the decrease in blood-adiponectin-concentration was suppressed by ingestion of the cheese produced using *Lactobacillus helveticus.*

**TABLE 6**

| Feed group | Blood-adiponectin-concentration (µg/ml) | | Change rate (%) |
|---|---|---|---|
| | Four weeks | Eight weeks | |
| Control food | 11.74 | 11.01 | 93.8 |
| Cheese food (Example 10) | 10.88 | 13.81 | 126.9 |

### EXAMPLE 14

### (Production of dog food)

Raw materials were mixed according to the formulation shown in Table 7 to produce a blood-adiponectin-concentration increase-acceleration and/or decrease-inhibition dog food according to the present invention.

**TABLE 7**

| | |
|---|---|
| SBT2171 cells | 2.5 (wt%) |
| Powdered skim milk | 13.5 |
| Soybean cake | 12.0 |
| Soybean oil | 4.0 |
| Corn oil | 2.0 |
| Palm oil | 27.0 |
| Corn starch | 14.0 |
| Flour | 9.0 |
| wheat bran | 2.0 |
| Vitamin mixture | 9.0 |
| Mineral mixture | 2.0 |
| Cellulose | 3.0 |

### EXAMPLE 15

### (Production of ATCC10386 cell powder)

A reconstituted skim milk medium (contains powdered skim milk: 13 wt%, yeast extract: 0.5 wt%) was sterilized at 95°C for 30 minutes. *Lactobacillus helveticus* ATCC10386 was then inoculated into the medium, and cultured at 37°C for 16 hours. The resulting culture was freeze-dried to obtain a culture powder of *Lactobacillus helveticus* ATCC10386. The culture powder can be used directly as the visceral fat accumulation inhibitor according to the present invention.

### EXAMPLE 16

### (Production of ATCC10797 cell powder)

A reconstituted skim milk medium (contains powdered skim milk: 13 wt%, yeast extract: 0.5 wt%) was sterilized at 95°C for 30 minutes. *Lactobacillus helveticus* ATCC10797 was then inoculated into the medium, and cultured at 37°C for 16 hours. The resulting culture was freeze-dried to obtain a culture powder of *Lactobacillus helveticus* ATCC10797. The culture powder can be used directly as the visceral fat accumulation inhibitor according to the present invention.

### EXAMPLE 17

### (Production of fermented milk)

*Lactobacillus helveticus* SBT2171 (FERM P-14381) was cultured at 37°C for 12 hours using 100 g of skim milk, inoculated into a fresh medium of the same kind (3 kg), and cultured at 37°C for 12 hours. One hundred (100) kg of skim milk was fermented at 32°C for 20 hours using the resulting fermented milk as a starter to obtain fermented milk containing *Lactobacillus helveticus* SBT2171 (FERM P-14381) according to the present invention. The viable cell count of *Lactobacillus helveticus* SBT2171 (FERM P-14381) was 8.2×10⁸ cells/g. The fermented milk can be used directly as the visceral fat accumulation inhibitor according to the present invention.

### EXAMPLE 18

### (Production of SBT2171 cell powder)

Five (5) kg of water was added to 5 kg of a culture obtained in the same manner as in Example 17. The mixture was centrifuged (3500×g) for 20 minutes using a continuous centrifuge, and only cells were collected. After the addition of 1 kg of water, the mixture was centrifuged. This operation was repeated three times to remove non-cell components contained in the precipitate. Twenty (20) g of cells itself of *Lactobacillus helveticus* SBT2171 (FERM P-14381) was thus collected. The washed cells were freeze-dried to obtain a cell powder. The cell powder can be used directly as the visceral fat accumulation inhibitor according to the present invention.

### EXAMPLE 19

### (Production of SBT2171 cell powder tablet)

Four (4) parts of powdered skim milk was mixed with 1 part of the cell powder obtained in Example 18. The mixed powder was formed into tablets (1 g) by a normal method using a tableting machine to obtain cell powder tablets each containing 200 mg of the cells itself of *Lactobacillus helveticus* SBT2171 (FERM P-14381). The cell powder tablet can be used directly as the visceral fat accumulation inhibitor according to the present invention.

### EXAMPLE 20

### (Production of yogurt drink)

Four (4) kg of granulated sugar, 3 kg of water, and 0.15 kg of pectin were added to 43 kg of the fermented milk obtained in Example 17. The mixture was then homogenized to obtain 50 kg of a visceral fat accumulation-inhibition yogurt drink according to the present invention. The yogurt drink had a mild favorable flavor and a pH of 3.6. The viable cell count of *Lactobacillus helveticus* SBT2171 (FERM P-14381) was 4.6×10⁸ cells/g.

### EXAMPLE 21

### (Production of cheese)

Several types of low-fat hard natural cheese were produced using raw material milk modified such that the fat percentage of the resulting cheese was 12 to 30%. Specifically, the raw material milk, of which the fat percentage was adjusted, was subjected to plate pasteurization at 75°C for 15 seconds. After cooling the raw material milk to 30°C, calcium chloride (0.01%) was added. A commercially available lactic acid bacteria starter (manufactured by Christian Hansen) (0.7%) and *Lactobacillus helveticus* SBT2171 (FERM P-14381) (1%) were added to the raw material milk. After the addition of rennet (0.003%) to coagulate the milk, the resulting product was cut. The cut product was stirred until the pH reached 6.1 to 6.2, and whey was discharged to obtain curds. The curds were placed in a mold and then compressed. Salt was then added to the resulting product to obtain Gouda-type low-fat hard natural cheese. The natural cheese can be used directly as the visceral fat accumulation-inhibition food according to the present invention.

### [Test Example 4]

### (Check on visceral fat accumulation-inhibition effect)

A visceral fat accumulation-inhibition effect was checked by an animal experiment using *Lactobacillus helveticus*. Four-week-old Fischer male rats were used for the animal experiment (one group consists of eight rats). A feed containing *Lactobacillus helveticus* SBT2171 (FERM P-14381), a feed containing *Lactobacillus helveticus* ATCC10386, or a feed containing *Lactobacillus helveticus* ATCC10797 was fed to three groups (cell feed groups), and a feed that did not contain cells was fed to another group (control feed group). The control feed was fed to each group for four weeks. The control feed was then fed to the control feed group for four weeks, and the feed containing *Lactobacillus helveticus* was fed to the cell feed groups for four weeks. The rats were dissected in the eighth week to measure the amount of visceral fat (mesentery, circumference of testicle, circumference of renal, and posterior abdominal wall).
The results are shown in Table 8. As shown in Table 8, it was confirmed that the amounts of visceral fat of the cell feed groups were generally lower than that of the control feed group. Specifically, it was confirmed that visceral fat accumulation was suppressed by ingestion of *Lactobacillus helveticus.*

**TABLE 8**

| Accumulated amount of visceral fat | | | | | |
|---|---|---|---|---|---|
| Feed | Amount of visceral fat (g) | | | | Total amount of visceral fat (g) |
| | Mesentery | Circumference of testicle | Circumference of renal | Posterior abdominal wall | |
| Control food | 2.54 | 3.51 | 0.94 | 2.15 | 9.14 |
| SBT2171 cell food | 2.04 | 3.31 | 0.91 | 2.07 | 8.33 |
| ATCC10386 cell food | 2.14 | 3.28 | 0.88 | 2.10 | 8.40 |
| ATCC10797 cell food | 2.22 | 3.56 | 0.89 | 2.11 | 8.78 |

### [Test Example 5]

### (Confirmation on visceral fat accumulation-inhibition effect)

A visceral fat accumulation-inhibition effect was confirmed using the cheese obtained in Example 21. Four-week-old Fischer male rats were used for an animal experiment (one group consists of eight rats). A feed containing the cheese was fed to one group (cheese feed group), and a feed that did not contain the cheese was fed to the other group (control feed group). The control feed contained milk casein as protein source and butter oil as a lipid source. The control feed was prepared so that the content of common components, main minerals, and vitamin E (α-tocopherol) was the same as that of the feed containing the cheese based on the component analysis results for the cheese (Table 5). In case of the cheese-containing feed, proteins and lipids derived exclusively from the cheese component. In the animal experiment, the rats were divided into two groups, and the control feed and the feed containing the cheese were respectively fed to the two groups. The rats were dissected in the eighth week to measure the amount of visceral fat (mesentery, circumference of testicle, circumference of renal, and posterior abdominal wall).
The results are shown in Table 9. As shown in Table 9, it was confirmed that the amount of visceral fat of the cheese feed group was generally lower than that of the control feed group. In particular, the amount of visceral fat in the mesentery of the cheese feed group was significantly lower than that of the control feed group. Specifically, it was confirmed that visceral fat accumulation (particularly mesentery) was suppressed by ingestion of the cheese produced using *Lactobacillus helveticus* SBT2171 (FERM P-14381).

**TABLE 9**

| Feed | Amount of visceral fat (g) | | | | Total amount of visceral fat (g) |
|---|---|---|---|---|---|
| | Mesentery | Circumference of testicle | Circumference of renal | Posterior abdominal wall | |
| Control food | 2.96 | 3.68 | 0.98 | 2.56 | 10.18 |
| Cheese food | 2.19 | 3.22 | 0.74 | 2.11 | 8.26 |
| (Example 21) | | | | | |

### EXAMPLE 22

### (Production of capsule)

Raw materials were mixed according to the formulation shown in Table 10 and then granulated. A capsule was charged with the resulting product to obtain a capsule having a visceral fat accumulation-inhibition effect.

**TABLE 10**

| | |
|---|---|
| SBT2171 cells (Example 18) | 20.0 (wt%) |
| Lactose | 24.5 |
| Soluble starch | 55.0 |
| Magnesium stearate | 0.5 |

### EXAMPLE 23

### (Production of drink)

Raw materials were mixed according to the formulation shown in Table 11. After charging a container with the mixture, the mixture was sterilized by heating to obtain a visceral fat accumulation-inhibition drink according to the present invention.

**TABLE 11**

| | |
|---|---|
| SBT2171 cells (Example 18) | 2.5 (wt%) |
| Sugar | 7.5 |
| Citric acid | 0.6 |
| Apple juice | 10.0 |
| Water | 79.4 |

## Claims

1. A blood-adiponectin-concentration increase-acceleration and/or decrease-inhibition agent comprising a culture and/or cells itself of lactic acid bacteria of the genus *Lactobacillus* as an active ingredient.

2. The blood-adiponectin-concentration increase-acceleration and/or decrease-inhibition agent according to claim 1, wherein the lactic acid bacteria of the genus *Lactobacillus* are *Lactobacillus gasseri* or *Lactobacillus helveticus.*

3. A blood-adiponectin-concentration increase-acceleration and/or decrease-inhibition food or drink comprising a culture and/or cells itself of lactic acid bacteria of the genus *Lactobacillus* as an active ingredient.

4. The blood-adiponectin-concentration increase-acceleration and/or decrease-inhibition food or drink according to claim 3, wherein the lactic acid bacteria of the genus *Lactobacillus* are *Lactobacillus gasseri* or *Lactobacillus helveticus.*

5. A blood-adiponectin-concentration increase-acceleration and/or decrease-inhibition feed comprising a culture and/or cells itself of lactic acid bacteria of the genus *Lactobacillus* as an active ingredient.

6. The blood-adiponectin-concentration increase-acceleration and/or decrease-inhibition feed according to claim 5, wherein the lactic acid bacteria of the genus *Lactobacillus* are *Lactobacillus gasseri* or *Lactobacillus helveticus.*

7. A visceral fat accumulation inhibitor comprising a culture and/or cells itself of *Lactobacillus helveticus* as an active ingredient.

8. A visceral fat accumulation-inhibition food or drink comprising a culture and/or cells itself of *Lactobacillus helveticus* as an active ingredient.
